# EUROPEAN PATENT SPECIFICATION

(11) **EP 2 072 515 B1**
(45) Date of publication and mention of the grant of the patent: **31.08.2011**
(21) Application number: 07450235.2
(22) Date of filing: 19.12.2007
(51) Int. Cl.: C07D 455/02, C07D 405/06, A61K 31/4375, A61P 3/04

(54) **Substituted quinazolidines for antidiabetic treatment**
Substituierte Quinazolidine zur antidiabetischen Behandlung
Quinazolidines substituées pour traitement antidiabétique

(43) Date of publication of application: 24.06.2009
(73) Proprietor: 55pharma Drug Discovery & Development AG, 1010 Wien (AT)
(72) Inventor: Adorjan, Immanuel, 3430 Tulln (AT); Bauer, Leonhardt, 1010 Wien (AT); Frobel, Klaus, 42113 Wuppertal (DE); Fürnsinn, Clemens, 1020 Wien (AT)
(74) Representative: Schwarz, Albin

(56) References cited:
- WO-A-2007/050802

## Description

### FIELD OF.THE INVENTION

This invention relates to the use of novel compounds represented by the formula I below for treatment and/or prevention of diabetes mellitus and its complications, for treatment and/or prevention of hyperlipidemia, for treatment of diabetic dyslipidemia, for treatment and/or prevention of the metabolic syndrome, for treatment of diseases related to metabolic dysfunction, for treatment of obesity or obesity-related diseases. The invention also includes pharmaceutical compositions and kits comprising these compounds alone or in combination with other drugs or compounds aiming towards an improved treatment or prevention of the aforementioned diseases or syndromes in humans and/or animals.

### BACKGROUND OF THE INVENTION

Diabetes mellitus is a chronic disease characterized by hyperglycaemia and deranged glucose metabolism. Hyperglycaemia results from either deficiency of the glucose-lowering hormone insulin or from resistance of peripheral tissues to the effects of insulin together with inadequate levels of insulin secretion to compensate. There are two main forms of diabetes: type 1 and type 2 diabetes mellitus. Type 1 diabetes is an autoimmune disease that results in the permanent destruction of insulin producing beta cells of the pancreas. Normally, type I diabetes manifests during adolescence and is life threatening unless treated with exogenous insulin via injection. Type 2 diabetes is a metabolic disorder that is primarily characterized by peripheral insulin resistance, relative insulin deficiency, and mild hyperglycaemia at onset. In contrast to type 1 diabetes, type 2 diabetes may go unnoticed for years before diagnosis. Risk factors of type 2 diabetes include obesity, age, first degree relatives with type 2 diabetes, history of gestational diabetes, hypertension and hypertriglyceridaemia. The most prevalent factors driving the development of insulin resistance and type 2 diabetes are life style associated, the main risk factor being obesity. Around 90% of the patients with type 2 diabetes are overweight or obese. Increased fat mass, especially an excess of abdominal fat causes insulin resistance, insulin resistance places a greater demand on the pancreatic beta-cells to produce insulin and due to exhaustion of the pancreas, insulin production declines with age leading to the development of apparent diabetes. In developed countries, type 2 diabetes represents about 90% of all diabetes.
Ref.: *Report of World Health Organisation: Definition and diagnosis of diabetes mellitus and intermediate hyperglycemia. WHO*/*IDF consultation, WHO, Geneva, 2006*
Diabetes mellitus is a growing health burden across the world. It is one of the most common diseases globally and among the leading causes of death in developed countries. At present, the three countries estimated to have the highest number of people with diabetes are India, China and the USA. Although the number of people with diabetes is already very high, numbers continue to increase at an alarming rate. The prevalence of diabetes worldwide is expected to double between 2000 and 2030 (2.8% in 2000 and minimum 4.4% in 2030). The total number of people with diabetes is projected to rise from 171 million in 2000 to at least 366 million in 2030 with the greatest relative increase anticipated in the developing countries in the Middle East, Africa and India. Although there is also a noticeable increase in type 1 diabetes, presumably due to changes in environmental risk factors, the "diabetes epidemic" is driven mainly by an increasing number of patients with type 2 diabetes. This is attributed to population growth, ageing, urbanisation and increased prevalence of obesity and physical inactivity. In some parts of the world overweight (Body Mass Index, BMI > 25) and obesity (BMI > 30) have increased to epidemic proportions in association with rapid cultural and social changes, including the excessive consumption of diets high in fat and protein. The human and economic costs of this epidemic are enormous. Weight-related escalating diabetes prevalence and cardiovascular disease, which is associated with diabetes, are expected to be the most significant public health concerns throughout this century and will lead to an immense financial burden. At present, the annual direct healthcare costs of diabetes are estimated to be at least between 153 and 286 billion dollars. In the light of such development, there is a big requirement for effective interventions including dietary and behavioural changes as well as pharmacological approaches.
Ref.: Zimmet P, Alberti KG M M, Shaw J: Global and societal implications of the diabetes epidemic. Nature 414, 782-787, 2001*;* Wild S, Roglic G, Green A, Sicree R, King H: Global prevalence of diabetes, estimates for the year 2000 and projections for 2030. Diabetes Care 27, 1017-1053, 2004

While established treatment regimens allow the diabetic patient an almost normal life for the short term, prolonged presence of the disease over time leads to serious damage of tissues, especially nerves and blood vessels. The resulting late complications of diabetes include coronary artery and peripheral vascular disease, cerebrovascular disease, diabetic neuropathy, diabetic foot, nephropathy and retinopathy. This causes cumulative proportions of disabilities and increased mortality. In virtually every developed society, diabetes is ranked among the leading causes of blindness, renal failure and lower limb amputation and about half of the money spent on diabetes care goes towards the costs of managing complications. The mechanisms by which diabetes leads to complications are not fully understood, but large studies have clearly confirmed that intensified therapy aiming at an early and stringent control of blood glucose reduces the incidence and severity of complications. Although early intense intervention increases the initial costs, the long term human and economic costs resulting from complications are decreased. This highlights the rationale not only for early lifestyle intervention but also for early pharmacotherapy and for the definition of ambitioned target levels of a near-normal control of blood glucose. As a consequence, any new drug or drug combination that contributes to further improvement and optimisation of blood glucose control is a valuable tool to prevent late complications and to reduce the medical and economic burden of diabetes.
Ref.: DCCT Research Group: The effect of intensive treatment of diabetes on the development and progression of long-term complications insulin-dependent diabetes mellitus, N Engl J Med 329, 977-986, 1993*;* UK Prospective Diabetes Study (UKPDS) Group: Intensive blood-glucose control with sulphonylureas or insulin compared with conventional treatment and risk of complications in patients with type 2 diabetes (UKPDS 33). Lancet 352, 837-853, 1998
UK Prospective Diabetes Study Group, UKPDS: Effect of intensive blood-glucose control with metformin on complications in overweight patients with type 2 diabetes (UKPDS 34). Lancet 352, 854-65, 1998

Both, type 1 and 2 diabetes mellitus have no medically proven cure and, hence, the main goal of treatment is the reduction of morbidity and mortality from complications. This can be achieved through effective treatment of hyperglycaemia with HbA_{1c} as a valuable readout parameter for glucose control over time. In type 1 diabetes, treatment with exogenous insulin is essential and, hence, improvement of blood glucose control is mainly reached by more sophisticated insulin injection regimens. Type 2 diabetes is a chronic, progressive disease and its pathophysiology varies markedly more among patients than that of type 1 diabetes. This suggests versatile strategies for prevention, diagnostic screening and treatments of type 2 diabetes. Besides lifestyle management, blood pressure control, cardiovascular risk protection and diabetic complications screening, pharmaceuticals are needed to optimise the treatment and outcome. In this context, a variety oral drugs is available for the treatment of type 2 diabetes. These drugs affect blood glucose via different mechanisms of action. According to the global guidelines for type 2 diabetes from the International Diabetes Foundation treatment recommendations are as follows: The insulin sensitising biguanide metformin is the drug of choice for first-line oral therapy of type 2 diabetes. Its major effect is to lower glycaemia by decreasing the hepatic glucose output. When metformin fails to sufficiently control blood glucose concentrations, sulfonylureas and/or PPARγ agonists should be added. Whereas sulfonylureas enhance insulin secretion, PPARγ agonists (thiazolidinediones) increase the sensitivity of muscle, fat, and liver to insulin. Further additional treatment options are α-glucosidase inhibitors, exenatide, glinides, or pramlintide. α-Glucosidase inhibitors reduce the rate of digestion of polysaccharides in the small intestine, which delays glucose absorption from the intestine and lowers postprandial plasma glucose concentrations. Glinides stimulate insulin secretion similar to sulfonylureas but with shorter half life. Exenatide (glucagon-like peptide 1 agonist) potentiates glucose mediated insulin secretion and pramlintide (amylin agonist) slows gastric emptying and inhibits glucagon production. If drugs and lifestyle-interventions are unable to maintain blood glucose control, insulin therapy is required at the late stage of the disease development.
Ref.: International Diabetes Foundation, Clinical Guidelines Task Force: Global guideline for type 2 diabetes, 2005. www. idf.org/webdata/docslIDF%20GGT2D.pdf; Nathan DM, Buse JB, Davidson MB, Heine RJ, Holman RR, Sherwin R, Zinman B: Management of hyperglycemia in type 2 diabetes: a consensus algorithm for the initiation and adjustment of therapy: a consensus statement from the American Diabetes Association and the European Association for the Study of Diabetes. Diabetes Care 29, 1963-1972, 2006

Apart from varying pathophysiology among patients, type 2 diabetes is a progressive disease with worsening glycaemia over time. Since monotherapies fail to reach glycaemic goals in almost three out of four patients, more than one medication will be necessary for the majority of patients over time and combinations of drugs with different mechanisms of action will encounter best treatment success in most cases. Nevertheless, numerous medications in several combinations still fail to achieve and maintain glycaemic levels to provide optimal health care status for most individual patients, which emphasises the continuing requirement for new and better drugs. Apart from unsatisfactory performance with respect to the treatment targets in glycaemic control, the prescription of many glucose lowering drugs is limited by concerns about adverse effects. Metformin, recommended for first-line oral therapy of type 2 diabetes, is relatively well tolerated. The most common adverse effects of metformin are gastrointestinal problems, but metformin has also been associated with lactic acidosis as an extremely rare but also an extremely dangerous adverse effect. Gastrointestinal problems are even much more common for other classes of drugs for type 2 diabetes. At least one third of the patients taking glucosidase inhibitors, exenatide or pramlintide are afflicted by gastrointestinal side effects, which are a frequent cause for discontinuation of treatment. Gastrointestinal effects are not a problem with sulfonylureas and glinides, but these drugs act by inducing insulin secretion and bare the risk of hypoglycaemia, which in extreme cases can be life threatening. And finally, the thiazolidinediones, which initially produced high expectations because of their favourable insulin sensitising mechanism of action, revealed to induce fluid retention and have recently even been suspected of increasing myocardial infarction and the risk of death from cardiovascular causes. Unsatisfactory efficacy in reaching the treatment goals, frequent problematic adverse effects and in many cases high costs are therefore unresolved problems in the present pharmaceutical treatment options for type 2 diabetes. Considering available pharmaceutical tools in the light of the alarming epidemiology of type 2 diabetes, an urgent need is obvious for new drugs with a better therapeutic index, i.e. with an improved relation of efficacy per adverse effects.
Ref.: Nathan DM, Buse JB, Davidson MB, Heine RJ, Holman RR, Sherwin R, Zinman B: Management of hyperglycemia in type 2 diabetes: a consensus algorithm for the initiation and adjustment of therapy: a consensus statement from the American Diabetes Association and the European Association for the Study of Diabetes. Diabetes Care 29, 1963-1972, 2006*;* Nissen SE, Wolski K: Effect of rosiglitazone on the risk of myocardial infarction and death from cardiovascular causes. N Engl J Med 356, 2457-2471, 2007

In search of novel glucose lowering agents, early preclinical examination and characterisation is usually based on the study of rodent strains witch metabolic deviations resembling the diabetic state. In such animals, glucose homeostasis is usually charged by postprandial glycaemia and by a glucose tolerance test (GTT), which determines the increase in blood glucose after administration of a glucose solution. In the GTT, glucose can be administered intravenously (IVGTT), intraperitoneally (IPGTT) or orally (OGTT), the latter being the most physiological approach. Rodents most frequently used as models for type 2 diabetes include such, in which increased glycaemia is due to a genetic defect, to dietary intervention or to the administration of toxic pharmacological agents. Each specific approach has advantages and limitations. Commonly used genetic models are rats and mice afflicted by a gene defect that causes overeating and severe obesity (e.g., ZDF rats, db/db mice). In these animals, very severe insulin resistance is the driving force behind the development of hyperglycaemia and, hence, they are very responsive to some agents that act via insulin sensitisation. This reasonably mimics the situation in extremely obese patients with type 2 diabetes, but the predominance of insulin resistance often makes it difficult to demonstrate in such models the glucose lowering action of drugs, which act via mechanisms other than insulin sensitisation. Other prevalently used models are rodents injected with agents that destroy insulin producing cells (streptozotocin, alloxan) and, if dosed appropriately, cause relative insulin deficiency. However, this model lacks the component of primary insulin resistance, which is a crucial characteristic of type 2 diabetes. Dietary models, in particular animals fed with a diet of very high fat content (high fat-diet, HFD) simulate better the pathogenesis of type 2 diabetes in the prevalent overweight patient. Since the degree of metabolic derangement remains limited, these models are comparable only with the early stages of the development of type 2 diabetes. There are strain differences regarding the extent of the HFD-induced derangement of glucose homeostasis with, e.g., C57/BL mice being more susceptible to HFD-induced metabolic derangements than other strains. The degree and the characteristics of the derangement can also be modulated by the diet composition. Usually, HFDs have a fat content around 60% (of calories) and contain carbohydrates and protein at a rate comparable to humans eating too much fat. Alternative HFDs are almost completely free of carbohydrates, which has the advantage of leading to more severe metabolic consequences within a shorter period of time, but mimics the situation in obese patients less appropriately.
Ref.: Surwit RS, Kuhn CM, Cochrane C, McCubbin JA, Feinglos MN: Diet-induced type II diabetes in C57BL/6J mice. Diabetes 37, 1163-1167, 1988*;* Winze/l MS, Ahrén B: The high-fat diet-fed mouse: a model for studying mechanisms and treatment of impaired glucose tolerance and type 2 diabetes. Diabetes 53 (Suppl 3), S215-219, 2004 Burcelin R, Crivelli V, Dacosta A, Roy-Tirelli A, Thorens B: Heterogeneous metabolic adaptation of C57BL/6J mice to high-fat diet. Am J Physiol 282, E834-E842, 2002

In WO 2007/050802 certain quinolizidines and octahydropyridopyrazines are disclosed as opioid receptor antagonists.

In summary, there is still an unfulfilled need for compounds, compound combinations and therapies that may be used to overcome the aforementioned set-backs of state of the art diabetic treatments. The present invention is directed to these, as well as other important ends.

### SUMMARY OF THE INVENTION

The present invention is generally directed to substituted quinazolidine derivatives, pharmaceutical compositions containing these compounds and methods of their pharmaceutical use.

The invention is directed to compounds of formula I: 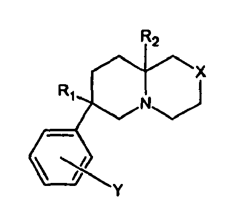
R¹: C₁-C₆ alkyl, Phenyl,
R²: H, C₁-C₆ alkyl, alkylcycloalkyl
X: Carbonyl, CR³R⁴, C(OH)R⁶ , CR⁶OR⁶
Y: (R³)ₖ where k=0,1,2,3
where R³ equals:
H, F, Cl, Br, CF₃, C₁-C₆ alkyl, cycloalkyl, phenyl,
O(CH₂)ₖ R⁵, N(CH₂)ₖ R⁵ where k=0,1,2,3
O(CH₂)ₙO(CH₂)ₖ R⁵, where n=1,2,3,4 , k=0,1,2,3
N(CH₂)ₙO(CH₂)ₖ R⁵ where n=2,3,4 , k=0,1,2,3
O(CH₂)ₙO(CH₂)ₘO(CH₂)ₖ R⁵ where n=1,2,3,4 , m=1,2,3,4, k=0,1,2,3 excluding m=n=1
N(CH₂)ₙO(CH₂)ₘO(CH₂)ₖ R⁵ where n=2,3,4 , m=1,2,3,4, k=0,1,2,3 excluding m=n=1 (CH₂)ₖ R⁵ where k=0,1,2,3
(CH₂)ₙO(CH₂)ₖ R⁵ where n=1,2,3,4 , k=0,1,2,3
(CH₂)ₙO(CH₂)ₘO(CH₂)ₖ R⁵ where n =1,2,3,4 , m=1,2,3,4, k=0,1,2,3 NHR⁵, N(R⁵)₂, NR4 (C=O) R⁵, NR⁴ (SO₂) R⁵
Piperidyl, pyrrolidinyl, morpholinyl,
where R⁴ equals: H, C₁-C₆ alkyl, cycloalkyl, alkylcykloalkyl
where R⁵ equals:
H, C₁-C₆ alkyl, isopropyl, isobutyl, cyclopentyl, cyclohexyl, cycloheptyl, benzyl, phenyl, 4-biphenyl, 1,3-dioxan-2-ylethyl, 2-methyl-2-phenylpropyl, 2-biphenyl, 4-biphenyl, 2-phenoxyphenyl, 4-phenoxyphenyl,
where R⁶ equals:
(CH₂)ₖ R⁵ where k=0,1,2,3
(CH₂)ₙO(CH₂)ₖ R⁵ where n=1,2,3,4 , k=0,1,2,3
(CH₂)ₙO(CH₂)ₘO(CH₂)ₖ R⁵ where n=1,2,3,4 , m=1,2,3,4, k=0,1,2,3
4-biphenyl, 1,3-dioxan-2-ylethyl, 2-methyl-2-phenylpropyl

### DESCRIPTION OF COMPOUNDS OF FORMULA I

The present invention is generally directed to substituted quinolizidine compounds, pharmaceutical compositions containing these compounds, and methods of their pharmaceutical use.

As used herein, the terms "stereoisomers" mean compounds that possess identical chemical constitution, but differ as in regard to the arrangement of the atoms or groups in space. As used herein, the term "partial stereoisomers" mean stereoisomers having two or more chiral centers wherein at least one of the chiral centers possesses defined stereochemistry. If not indicated otherwise, stereoisomeric mixtures may contain stereoisomers and/or partial stereoisomers in different relative quantitities, which may in itself be racemic or optically enriched.

Pharmaceutically acceptable salts include, but are not limited to, the conventional salts or the quaternary ammonium salts of the parent compound formed, e.g., from non-toxic inorganic or organic acids, which are for example, prepared by methods known in the art from common inorganic acids (e.g. hydrochloric, hydrobromic, phosphoric, nitric, sulfamic, sulphuric) or the salts prepared from organic acids (e.g. ascorbic, tartaric, citric, maleic, hydroxymaleic, fumaric, oxalic, acetic, propionic, succinic, toluenesulfonic, methanesulfonic, ethane disulfonic, stearic, palmeic, glycolic, lactic, malic, phenylacetic, sulfanilic, glutamic, benzoic, salicylic, 2-acetoxybenzoic, isothioilic).

The term "effective amount" means any amount of a product as described herein that may be therapeutically effective to prevent or treat the symptoms of a particular disease, disorder, or side effect which include, but are not limited to those pathological conditions associated with diabetes.

As used herein, the term "pharmaceutically acceptable" refers to those compounds, compositions, materials, and/or dosage forms that are, within the scope of sound medical judgment, suitable for contact with the tissues of animals and human beings without excessive irritation, toxicity, allergic response, or other complications or problems commensurate with a reasonable risk-benefit ratio. The term specifically encompasses veterinary uses.

As used herein, the term "dosage unit" means physically discrete units suited as unitary dosages for the treatment of the particular individual. Each unit may contain a predetermined quantity of active material as claimed in the invention calculated to produce the desired therapeutic effect(s) in association with an adequate pharmaceutical carrier. Dosage unit forms of the invention may be dictated by the particular therapeutic effect(s) to be achieved, the unique characteristics of the active compound(s), and the limitations inherent in the art of compounding such active compound(s).

The term "treatment" or "treating" as used herein includes, without limitation, preventive (e.g., prophylactic), curative or palliative treatment. As used herein, the term "patient" means animals, including mammals, preferably humans.

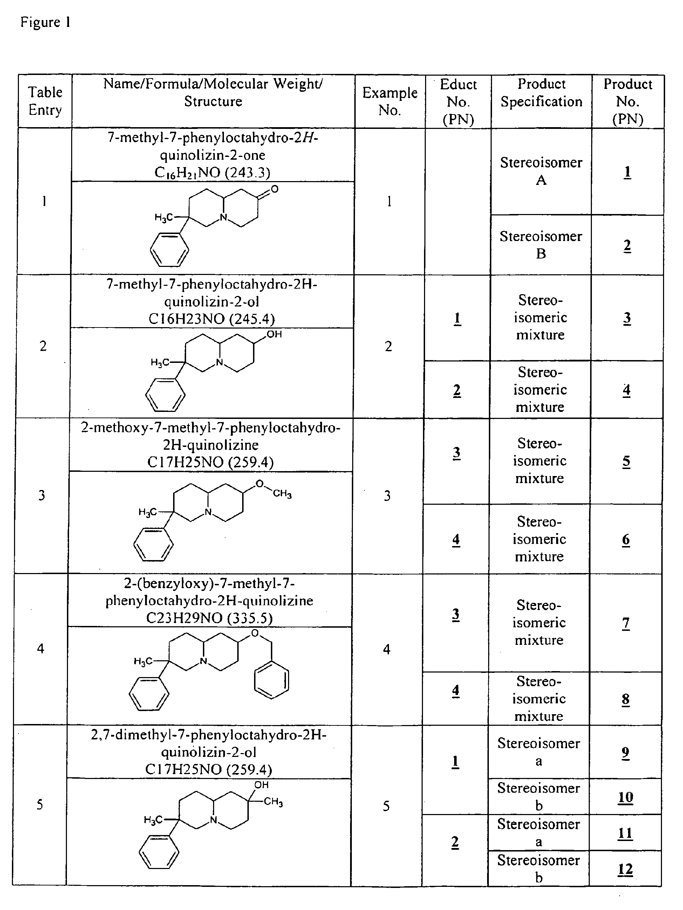

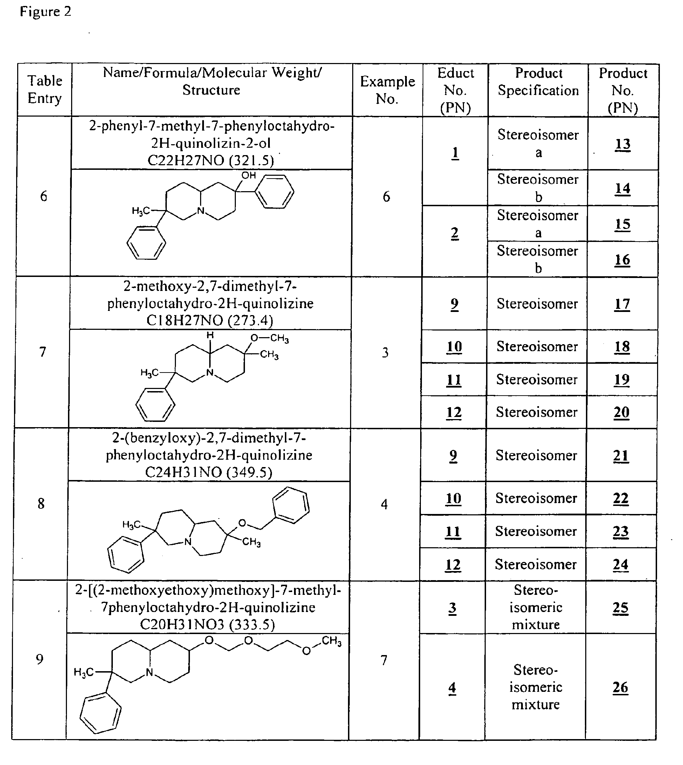

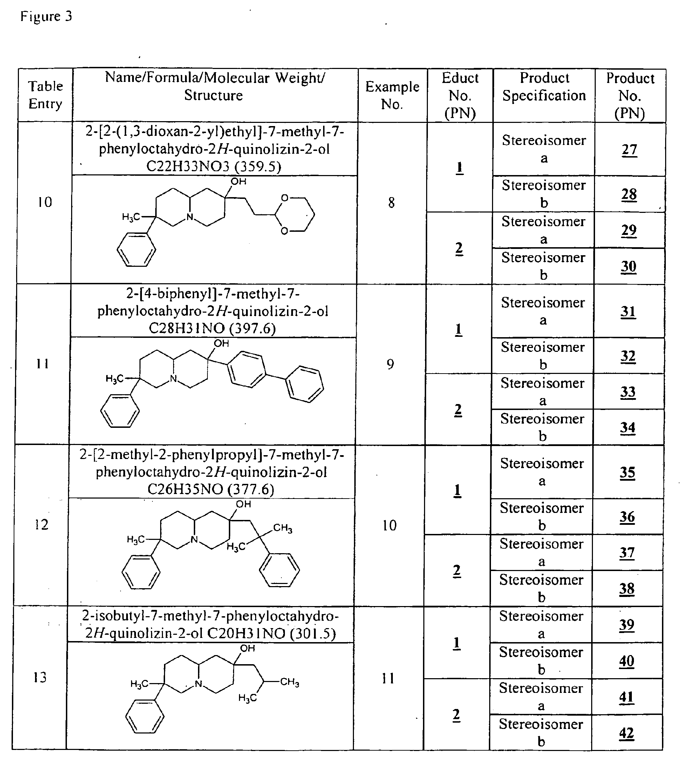

Preferred embodiments are listed in Figure 1-3.

Although the compounds of the present invention may be administered as pure chemicals, it is preferable to provide the active ingredient as a pharmaceutical composition, comprising an effective amount of one or more of the compounds of the invention, preferably one or more compounds of formula I, as described herein, together with one or more pharmaceutically acceptable carriers, i.e. in the sense of being compatible with the other ingredients of the composition and not deleterious to the recipient thereof.

The compounds of the invention may be administered in an effective amount by any of the standard techniques well-established in the medical field. The compounds employed in the methods of the present invention may be given by any means that results in the contact of the active ingredient(s) with the relevant site(s) of action in the body of a patient. The compounds may be administered by any standard means available for use in conjunction with pharmaceuticals, either as individual therapeutic agents or in a combination of therapeutic and/or preventive agents. For example, they may be administered as the sole active ingredients in a pharmaceutical composition, or they can be used in combination with other therapeutically active ingredients or with a pharmaceutically acceptable carrier in quantities, which may be determined by the solubility, the chemical nature, the route of administration or other means being advantageous for an effective treatment by the targeted therapy.

The dosage of the compounds of this invention that will be most suitable for prevention or treatment will vary with the particular compound used, the form of administration, and the physiological characteristics of the particular patient under treatment. Generally, small dosages may be used initially and, if required, increased by small increments until the desired effect under the respective circumstances is reached. Generally speaking, oral administration may require higher dosages than parenteral administrations. Although the proper dosage of the products of this invention will be readily ascertainable by one skilled in the art, once armed with the present disclosure, by way of general guidance, for example, typically a daily dosage of the compound of the invention, i.e., a compound of formula I, as described herein, may range from about 0.001 to about 500 milligrams per kilogram of patient body weight (and all combinations and subcombinations of ranges and specific dosage amounts therein). Preferably, the daily dosage may be about 0.01 to about 250 milligrams per kilogram of patient body weight of the compound of this invention, preferably a compound of formula I.

### METHODS OF PREPARATION

If not stated otherwise, the following materials and solvents have been used. HPLC: Acetonitril (ACN), Water (LC-MS grade, Fisher Scientific); Formic acid, puriss. p.a. (eluent additive for LC-MS, Fluka); Dry solvents for chemical reactions: N,N-dimethylformamide (DMF), puriss., absolute (over molecular sieve (H₂O ≤0.01%), ≥99.8% (GC), Fluka), diethyl ether (DEE), puriss., (dried over molecular sieve (H₂O ≤0.005%), ≥99.8% (GC), Fluka), tetrahydrofuran (THF), puriss., absolute (over molecular sieve (H₂O ≤0.005%), ≥99.5% (GC), Fluka)

If not stated otherwise, the following materials and solvents have been used for extraction and/or column chromatography: Petrol ether (PE): bp: 40-60°C, Baker reinst, (Baker); Ethyl acetate (EtOAc), Methanol (MeOH), Diethyl ether (Et₂O): GPR Reactapur (VWR Prolabo); Dichloromethane (CH₂Cl₂): for synthesis (Merck Darmstadt); Toluene (Tol): Baker analyzed, (Baker); Ethanol (EtOH): absolute, 99.9% (Australco)

If not stated otherwise, the following reagents have been used for chemical reactions: 3-Chloropropionaldehyde diethylacetal, tech.(>90%(GC), Fluka); 1-Methylbenzyl cyanide (96%, Aldrich), Sodium hydride (NaH), 60% dispersion in mineral oil (Aldrich); Lithiumaluminiumhydride (LAH), reagent grade, 95%, powder (Aldrich); Sulfuric acid (H₂SO₄), 95-97%, Baker analyzed (Baker, was diluted with tab-water to used concentration); Sodium hydroxide (NaOH), Baker analyzed, (Baker); 3-Buten-2-one (methyl vinyl ketone), 99%, (Aldrich); Hydrochloric acid (HCl), 37-38%, Baker analyzed (Baker, was diluted with tab-water to used concentration); Methyllithium (MeLi) solution, purum, ∼1 M in cumene/THF (Aldrich), Phenyllithium (PhLi) solution, ∼1.9 M in butyl ether (Fluka); Jodmethane (MeI), purum: >99.0% (GC) (Fluka), Benzyl chloride (BnCl), puriss.:>99.5% (GC) (Fluka); Sodium sulfate (Na₂SO₄), p.a., ACS, ISO, anhydrous (Roth); Magnesium sulfate anhydrous (MgSO₄), puriss. p.a., drying agent, >98% (KT) (Fiuka); Ammonium chloride (NH₄Cl), purum p.a., >99% (Fluka); Sodium carbonate (NaCO₃), purum, >98.0% (T) (Fluka); Ninhydrin, 97% (Aldrich); 2-Methyl-2-phenylpropylmagnesium chloride solution 0.5 M in diethyl ether (Aldrich); Isobutylmagnesium chloride solution 2.0 M in diethyl ether(Aldrich); (1,3-Dioxan-2-ylethyl)magnesium bromide solution 0.5 M in tetrahydrofuran (Aldrich); 4-Biphenylmagnesium bromide solution 0.5 M in tetrahydrofuran (Aldrich); 2-Methoxyethoxymethyl chloride (MEMCl), technical grade (Aldrich); Triethylamine (TEA), puriss. p.a., ≥99.5% (GC) (Aldrich)

If not stated otherwise reaction mixtures were purified by Column Chromatography (CC) on Silica gel (silica gel 60, 0.06-0.2 mm, Roth) following common procedures. In CC method A a gradient of CH₂Cl₂/MeOH was used, in CC method B a gradient of PE/EtOAc was used, in CC method C a gradient of PE/EtOAc with 1% TEA was used. Eluates were examined by Thin Layer Chromatography (TLC), unified as single spot products or defined stereoisomeric mixtures and evaporated to dryness under reduced pressure (bath temperature 20-40 °C); TLC plates: TLC silica gel 60 F₂₅₄ glass plates 20*20 cm Multiformat pre-scored to 5*10 cm (Merck); detection by UV and/or staining with Ninhydrin solution (0.2 g in 100 mL ethanol, heat).

If not stated otherwise, the reaction products were identified and/or characterized by HPLC/MS using the following set-up: Instrumentation: SCL-10AVP, controller; DGU-20A5, degasser, FCV-10ALVP, low pressure gradient mixing unit, LC-10ADVP pump, SIL10AP, autosampler with 500 µl syringe and 400 µl injection loop, SPD-M10AVP, PDA detector, LCMS 2010A MS detector (Shimadzu); SmartMix, gradient mixer with 350 µl mixing chamber (Knauer); N₂ LCMS 1, nitrogen generator (Claind); E2M28, two stage rotary vacuum pump (Edwards); Column: Synergi 4µ Fusion-RP 80A 150x2.0 mm, with Security Guard Cartridge Fusion-RP 4 x 2.0 mm (Phenomenex Inc.); Software: LabSolutions - LCMSolution Ver. 3.41 (Shimadzu); Sample preparation: Samples were weighted, dissolved in acetonitril, and diluted to a final volume of 1 ml with a concentration of 0.5-0.05 mg/ml in acetonitril/water (with 0.1% formic acid) = 9:1. The injection volume was adjusted (1 - 10 µl) to achieve an injection of 0.5 µg sample. Solvents: solvent A: water with 0.1 % formic acid, solvent B: acetonitril with 0.1% formic acid.

Reaction products and stereoisomers were characterised by HPLC/MS via relative retention time in minutes after injection (RTT) applying the following methods as mentioned in the examples below. Detected ions are given in intensities in percent relative to base peak (100%). HPLC/MS Method A: flow: 0.5 ml/min; linear gradient (%A is the difference to 100%): start at 10% B, in 10 min to 50% B, then in 2 min to 100% B, then kept for 10 min at 100% B, then in 3 min to 10% B, then 10 min equilibration at 10% B; total run time: 35 min; PDA detector: wavelength: 190 - 600 nm, sampling rate: 1.56 Hz, MS detector: ionization mode: ESI positive, mass range: 150 - 500 ± 0.5 m/z; scan speed: 500 amu/sec; detector voltage: 1.25 kV; heat block temperature: 200 °C: CDL temperature: 250 °C; nebulizing gas flow: 1.5 L/min; dry gas pressure: 0.1 MPa.

If not stated otherwise RT stands for room temperature or ambient temperature, which typically lies between 20 and 25 °C.

### EXAMPLES

### EXAMPLE 1: Preparation of 1 and 2 following the procedure of Frank D. King, J Chem. Soc. Perkin Trans. 1, 447-453 (1986).

To a suspension of dry DMF (100 ml) and sodium hydride, 60% (4.1 g, 0.17 mol) at 70 °C in a dry and inert atmosphere (Argon) methylphenylacetonitrile (10 ml, 0.075 mol) was added. After stirring at 70 °C for 1 h. 3-chloropropionaldehyde diethyl acetal (13.2 g, 0.079 mol) was added drop wise. After stirring at 70 °C for 1 h and cooling to RT, the reaction mixture was poured into 1 L of ice-water. The product was extracted with Et₂O (3 x 200 ml). The combined Et₂O extracts were filtered over Na₂SO₄ and *evaporated in vac.* to dryness yielding about 19.1 g of crude product which was directly used in the next step. To a suspension of dry THF (115ml) and LAH (2.43 g, 0.065 mol) in a dry and inert atmosphere (Argon) concentrated sulphuric acid (1.6 ml, 0.03 mol) was added drop wise under cooling with ice water. After stirring at 0 °C for 1h, a solution of the crude product from the previous step (19.1 g, 0.073 mol) in dry THF (19 ml) was added drop wise and the reaction mixture stirred at RT for 5 h. After cooling to 0 °C I M NaOH (11.3 ml) was added, the formed precipitate was removed by suction filtration and Et₂O was used to wash the precipitate. The filtrate was evaporated *in vac.* to dryness yielding about 16 g crude product, which was dissolved in 65 ml Et₂O. Methylvinylketone (6.1 ml, 0.073 mol) was added and the reaction mixture was stirred at RT for 2 h. The reaction mixture was added drop wise to 350 ml of 2.5 M HCl. The phases were separated with a separator funnel, the aqueous phase was taken and refluxed for 2h. After addition of ice cubes, the mixture was neutralized with solid sodium carbonate and extracted with CH₂Cl₂. The combined organic layers were dried over Na₂SO₄ and the solvent was removed *in vac.* to dryness. The crude product (11.2 g) was purified by CC method B. yielding **1** (2.9 g) and **2** (5.1 g).
HPLC/MS Method A: **1**: RTT = 2.7 [ms: 262.1 (M+H₃O⁺), 244.1 (35%, M+H⁺)]; **2**: RTT =3.6 [ms: 262.1 (M+H₃O⁺), 244.1 (25%, M+H⁺)]

### EXAMPLE 2: Preparation of products 3 and 4

Product **1** or **2**, respectively, dissolved in dry DEE was added slowly to a stirred suspension of 1eq. LAH in dry DEE in a dry and inert atmosphere (Argon) at RT. After 2 h the reaction mixture was quenched with water, rendered alkaline with NaOH and extracted three times with Et₂O. The combined organic layers were dried over Na₂SO₄, filtered and *evaporated in vac.* to dryness yielding **3** or **4**, as mixtures of stereoisomers.
HPLC/MS Method A: **3**: Stereoisomer I: RTT = 2.9 [ms: 246.1 (M+H⁺)], Stereoisomer II: RTT = 3.4 [ms: 246.1 (M+H⁺)]; **4**: Stereoisomer I: RTT = 3.9 [ms: 246.1 (M+H⁺)], Stereoisomer II: RTT = 4.6 [ms: 246.1 (M+H⁺)]

### EXAMPLE 3: Preparation of products 5, 6, 17 to 20

Product **3**, **4, 9** to **12**, respectively, was dissolved in dry DMF, 2eq. NaH were added and stirred at RT for 45 min. 1.2 to 5eq. Mel were added and the reaction mixture was stirred over night. The reaction mixture was quenched with water and extracted with CH₂Cl₂. The combined organic phases were dried over MgSO₄, filtered and evaporated *in vac.* to dryness. CC method B delivered **5** and **6** as mixtures of stereoisomers and **17** to **20** as pure products.
HPLC/MS Method A: **5**: Stereoisomer I: RTT = 4.6 [ms: 260.1 (M+H⁺)], Stereoisomer II: RTT = 6.2 [ms: 260.1 (M+H⁺)]; **6**: Stereoisomer 1: RTT = 7.1 [ms: 260.1 (M+H⁺)], Stereoisomer II: RTT = 8.4 [ms: 260.1 (M+H⁺)); **17**: RTT = 6.0 [ms: 274.1 (M+H⁺), 242.1 (4%, M+H⁺-MeOH)]; **18**: RTT = 7.7 [ms: 274.1 (M+H⁺)]; **20**: RTT = 8.9 [ms: 274.1 (M+H⁺)]

### EXAMPLE 4: Preparation of products 7, 8, 21 to 24

Product **3, 4, 9** to **12,** respectively, was dissolved in dry DMF, 2eq. NaH were added and stirred at RT for 45 min. 1.2 to 5eq. BnCl were added and the reaction mixture was stirred over night. The reaction mixture was quenched with water and extracted with Et₂O. The combined organic phases were dried over MgSO₄, filtered and *evaporated in vac.* to dryness. CC method B delivered **7** and **8** as mixtures of stereoisomers and **21** to **24** as pure products.
HPLC/MS Method A: **7**: Stereoisomer I: RTT = 10.7 [ms: 336.1 (M+H⁺)], Stereoisomer II: RTT = 11.4 [ms: 336.1 (M+H⁺)]; **8**: Stereoisomer 1: RTT = 11.3 [ms: 336.1 (M+H⁺)], Stereoisomer II: RTT = 12.2 [ms: 336.1 (M+H⁺)]; **21**: RTT = 11.3 [ms: 350.1 (M+H⁺), 242.1 (4%, M+H⁺-BnOH)]; **23**: RTT = 11.6 [ms: 350.1 (M+H⁺), 242.1 (3%, M+H⁺-BnOH)]; **24**: RTT = 12.3 [ms: 350.1 (M+H⁺)]

### EXAMPLE 5: Preparation of products 9 to 12

Product **1** or **2**, respectively, was dissolved in a dry inert atmosphere (Argon) in dry DEE and cooled to -70 °C. 2eq. MeLi were added and kept for 1 h at -70 °C without stirring. After warming to RT over a period of 1 h the reaction mixture was hydrolyzed with aq. NH₄Cl solution and extracted with Et₂O. The organic phase was dried over MgSO₄, filtered and evaporated *in vac.* to dryness. Purification of the crude product by CC method A delivered **9** and **10** or **11** and **12** as pure products.
HPLC/MS Method A: **9**: RTT = 3.3 [ms: 260.1 (M+H⁺), 242.1 (4%, M+H⁺-H₂O)]; **10**: RTT = 4.5 [ms: 260.1 (M+H⁺)]; **11**: RTT =.5.1 [ms: 260.1 (M+H⁺),242.1 (2%, M+H⁺-H₂O)]; **12**: RTT = 5.6 [ms: 260.1 (M+H⁺)]

### EXAMPLE 6: Preparation of products 13 to 16

Product **1** or **2**, respectively, was dissolved in a dry inert atmosphere (Argon) in dry DEE and cooled to -70 °C. 2eq. PhLi were added and kept for 1 h at -70 °C without stirring. After warming to RT over a period of 1 h the reaction mixture was hydrolyzed with aq. NH₄Cl solution and extracted with Et₂O. The organic phase was dried over MgSO₄, filtered and evaporated *in vac.* to dryness. Purification of the crude product by CC method A delivered **13** and **14** or **15** and **16** as pure products.
HPLC/MS Method A: **13**: RTT = 9.9 [ms: 322.1 (M+H⁺)]; **14**: RTT = 8.9 [ms: 322.1 (M+H⁺), 304.1 (7%. M+H⁺-H₂O)]

### EXAMPLE 7: Preparation of products 25 or 26

Product **3** or **4**, respectively, was dissolved in dry DMF, 2eq. NaH were added and stirred at RT for 45 min. 1.2 to 5eq. MEMCl were added and the reaction mixture was stirred over night. The reaction mixture was quenched with water and extracted with CH₂Cl₂. The combined organic phases were dried over MgSO₄, filtered and *evaporated in vac.* to dryness. CC method C delivered **25** or **26** as mixtures of stereoisomers.
HPLC/MS Method A: **25**: Stereoisomer I: RTT = 7.8 [ms: 334.1 (M+H⁺)], Stereoisomer II: RTT = 8.4 [ms: 334.1 (M+H⁺)]; **26**: Stereoisomer I: RTT = 8.7 [ms: 334.1 (M+H⁺)], Stereoisomer II: RTT = 9.5 [ms: 334.1 (M+H⁺)]

### EXAMPLE 8: Preparation of products 27 to 30

Product **1** or **2**, respectively, was dissolved in dry DEE, 1.2eq. (1,3-dioxan-2-ylethyl)magnesium bromide solution was added and stirred at RT for 1.5 h. The reaction mixture was quenched with NH₄Cl solution and extracted with Et₂O. The combined organic phases were dried over MgSO₄, filtered and evaporated *in vac.* to dryness. CC method B delivered **27** to **30** as pure products.
HPLC/MS Method A: **29**: RTT = 8.7 [ms: 360.2 (M+H⁺)]; **30**: RTT = 9.0 [ms: 360.2 (M+H⁺), 342.2 (4%, M+H⁺-H₂O)]

### EXAMPLE 9: Preparation of products 31 to 34

Product **1** or **2**, respectively, was dissolved in dry DEE, 1.2eq. 4-biphenylmagnesium bromide solution was added and stirred at RT for 1.5 h. The reaction mixture was quenched with NH₄Cl solution and extracted with Et₂O. The combined organic phases were dried over MgSO₄, filtered and evaporated *in vac.* to dryness. CC method B delivered **31** to **34** as pure products.
HPLC/MS Method A: **33**: RTT = 12.5 [ms: 398.1 (M+H⁺)]; **34**: RTT = 12.3 [ms: 398.1 (M+H⁺), 380.1 (19%, M+H⁺-H₂O)]

### EXAMPLE 10: Preparation of products 35 to 38

Product **1** or **2**, respectively, was dissolved in dry DEE, 1.2eq. 2-methyl-2-phenylpropylmagnesium chloride solution was added and stirred at RT for 1.5 h. The reaction mixture was quenched with NH₄Cl solution and extracted with Et₂O. The combined organic phases were dried over MgSO₄, filtered and *evaporated in vac.* to dryness. CC method B delivered **35** to **38** as pure products.
HPLC/MS Method A: **37**: RTT = 12.1 [ms: 378.2 (M+H⁺)]; **38**: RTT = 12.3 [ms: 378.2 (M+H⁺), 360.2 (6%, M+H⁺-H₂O)]

### EXAMPLE 11 : Preparation of products 39 to 42

Product **1** or **2**, respectively, was dissolved in dry DEE, 1.2eq. isobutylmagnesium chloride solution was added and stirred at RT for 1.5 h. The reaction mixture was quenched with NH₄Cl solution and extracted with Et₂O. The combined organic phases were dried over MgSO₄, filtered and evaporated *in vac.* to dryness. CC method B delivered **39** to **42** as pure products.
HPLC/MS Method A: **41**: RTT = 9.9 [ms: 302.2 (M+H⁺)]; **42**: RTT = 10.3 [ms: 302.2 (M+H⁺), 284.2 (11%, M+H⁺-H₂O)]

### BIOLOGICAL METHODS

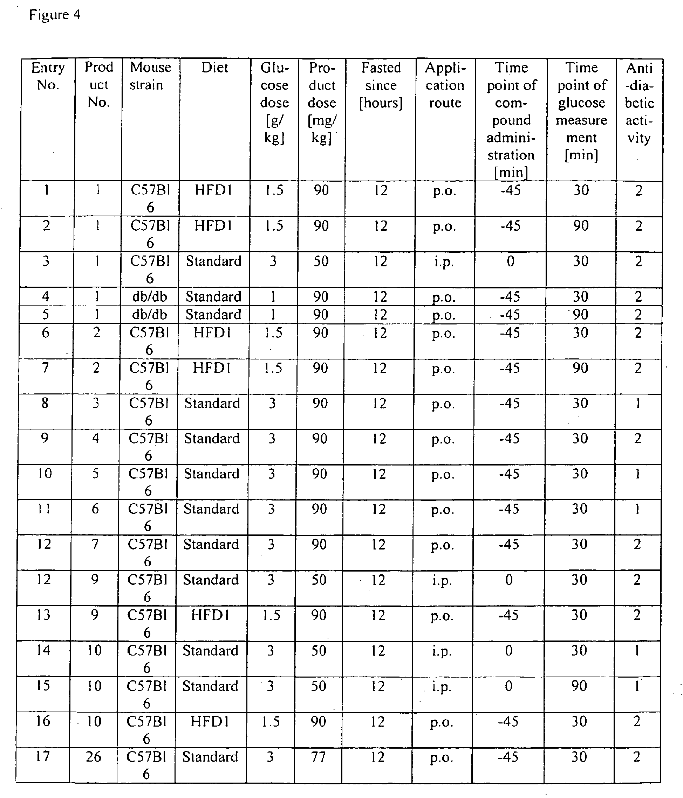

All animal experiments described below and listed in Figure 4 were performed in accordance to Austrian law and the principles of good laboratory animal care.

Data shown in Figure 4 are obtained using commercially available mice purchased, e.g. from the breeding facilities of the Medical University of Vienna (Austria) or Charles River Lab. (Belgium). Male mice were used at an age of 7 - 30 weeks and had free access to food and water except for defined fasting periods before experimentation. They were maintained at room temperature and a 12h light-dark cycle. Mice were fasted for 12 hours prior to oral glucose tolerance testing.

The antidiabetic activity of the products of formula I were evaluated in an oral glucose tolerance test in mice, in analogy to the procedure known to the general physician. Each mouse was either treated per os via gavage or injected intraperitoneally as specified in Figure 4 followed by oral administration via gavage of a glucose solution (1-3 g/kg as specified) at T=0 min. Blood was collected via puncture of the tip of the tail immediately before administration of products of formula I, immediately before administration of glucose, and at T=30 min and/or T=90 as the case may be. Blood glucose was determined using portable glucometers as commonly used in human diabetes.

The increment in blood glucose at T=min over levels measured at T=0 min was calculated for each animal. Mean values of the increment for treatment group and vehicle group were compared (typical group size n=6-10 mice). Percent reduction induced by products of formula I was the readout parameter for glucose lowering activity. As listed in Figure 4, an effect of 1 means a reduction of 15-30% of incremental blood glucose at the given time point T=min versus vehicle group, an effect of 2 means a reduction of more than 30% of incremental blood glucose at the given time point T=min versus vehicle group. The statistical significance of differences was assessed by standard methodologies, for example 2-tailed unpaired Student's T test; p<0.05 was considered statistically significant.

For biological testing, products of formula I were dissolved or suspended in 0.5% carboxymethylcellulosis containing 1-2% acetic acid. The vehicle group received the same amount of a 0.5% carboxymethylcellulosis solution containing 1-2% acetic acid.

Mice were permanently held on a standard laboratory chow diet (kg/kg: <10% crude fat) or on a high fat diet 1 (HFD1; energy content: 72 % fat, <1 % carbohydrate) as specified. Experiments with mice on a high fat diet were performed after a pre-feeding period of 5-6 weeks on HFD1.

Antidiabetic effects of products are listed in Figure 4 as evaluated in a glucose tolerance assay in mice.

## Claims

1. Compounds of formula I: 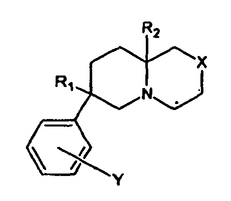
R¹: C₁-C₆ alkyl, Phenyl,
R²: H, C₁-C₆ alkyl, alkylcycloalkyl
X: Carbonyl, CR³R⁴, C(OH)R⁶, CR⁶OR⁶
Y: (R³)ₖ where k=0,1,2,3
where R³ equals:
H, F, Cl, Br, CF₃, C₁-C₆ alkyl, cycloalkyl, phenyl,
O(CH₂)ₖ R⁵, N(CH₂)ₖ R⁵ where k=0,1,2,3
O(CH₂)ₙO(CH₂)ₖ R⁵, where n=1,2,3,4 , k=0,1,2,3
N(CH₂)ₙO(CH₂)ₖ R⁵ where n=2,3,4, k=0,1,2,3
O(CH₂)ₙO(CH₂)ₘO(CH₂)ₖ R⁵ where n=1,2,3,4 , m=1,2,3,4, k=0,1,2,3 excluding m=n=1
N(CH₂)ₙO(CH₂)ₘO(CH₂)ₖ R⁵ where n=2,3,4 , m=1,2,3,4, k=0,1,2,3 excluding m=n=1
(CH₂)ₖ R⁵ where k=0,1,2,3
(CH₂)ₙO(CH₂)ₖ R⁵ where n=1,2,3,4 , k=0,1,2,3
(CH₂)ₙO(CH₂)ₘO(CH₂)ₖ R⁵ where n =1,2,3,4 , m=1,2,3,4, k=0,1,2,3
NHR⁵, N(R⁵)₂, NR4 (C=O) R⁵, NR⁴ (SO₂) R⁵
Piperidyl, pyrrolidinyl, morpholinyl,
where R⁴ equals: H, C₁-C₆ alkyl, cycloalkyl, alkylcykloalkyl
where R⁵ equals:
H, C₁-C₆ alkyl, isopropyl, isobutyl, cyclopentyl, cyclohexyl, cycloheptyl, benzyl, phenyl, 4-biphenyl, 1,3-dioxan-2-ylethyl, 2-methyl-2-phenylpropyl, 2-biphenyl, 4-biphenyl, 2-phenoxyphenyl, 4-phenoxyphenyl,
where R⁶ equals:
(CH₂)ₖ R⁵ where k=0,1,2,3
(CH₂)ₙO(CH₂)ₖ R⁵ where n=1,2,3,4 , k=0,1,2,3
(CH₂)ₙO(CH₂)ₘO(CH₂)ₖ R⁵ where n=1,2,3,4, m=1,2,3,4, k=0,1,2,3
4-biphenyl, 1,3-dioxan-2-ylethyl, 2-methyl-2-phenylpropyl

2. Pharmaceutical composition containing a compound of formula I as drug substance.

## Patentansprüche

1. Verbindungen der Formal I: 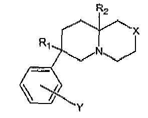 worin
R¹; C₁-C₆ Alkyl, Phenyl,
R²: H, C₁-C₆ Alkyl, Alkylcycloalkyl,
X: Carbonyl, CR³R⁴, C(OH)R⁶, CR⁶OR⁶,
Y: (R³)ₖ, worin k=0,1,2,3,
worin R³ bedeutet:
H, F, Cl, Br, CF₃, C₁-C₆ Alkyl, Cycloalkyl, Phenyl,
O(CH₂)ₖ R⁵, N(CH₂)ₖ R⁵, worin k=0,1,2,3,
O(CH₂)ₙO(CH₂)ₖ R⁵, worin n=1,2,3,4 , k=0,1,2,3,
N(CH₂)ₙO(CH₂)ₖ R⁵, worin n=2,3,4 , k=0,1,2,3,
O(CH₂)ₙO(CH₂)ₘO(CH₂)ₖ R⁵, worin n=1,2,3,4 , m=1,2,3,4, k=0,1,2,3, worin m=n=1 ausgeschlossen ist,
N(CH₂)ₙO(CH₂)ₘO(CH₂)ₖ R⁵, worin n=2,3,4 , m=1,2,3,4, k=0,1,2,3, worin m=n=1 ausgeschlossen ist,
(CH₂)ₖ R⁵, worin k=0,1,2,3,
(CH₂)ₙO(CH₂)ₖ R⁵, worin n=1,2,3,4 , k=D,1,2,3,
(CH₂)ₙO(CH₂)ₘO(CH₂)ₖ R⁵, worin n =1,2,3,4 , m=1,2,3,4, k=0,1,2,3,
NHR⁵, N(R⁵)₂, NR⁴(C=O)R⁵, NR⁴(SO₂)R⁵,
Piperidyl, Pyrrolidinyl, Morpholinyl,
worin R⁴ bedeutet: H, C₁-C₆ Alkyl, Cycloalkyl, Alkylcykloalkyl,
worin R⁵ bedeutet:
H, C₁-C₆ Alkyl, Isopropyl, Isobutyl, Cyclopentyl, Cyclohexyl, Cycloheptyl, Benzyl, Phenyl, 4-Biphenyl, 1,3-Ddioxan-2-ylethyl, 2-Methyl-2-phenylpropyl, 2-biphenyl, 4-Biphenyl, 2-Phenoxyphenyl, 4-Phenoxyphenyl,
worin R⁶ bedeutet:
(CH₂)ₖ R⁵, worin k=0,1,2,3,
(CH₂)ₙO(CH₂)ₖ R⁵, worin n=1,2,3,4 , k=0,1,2,3,
(CH₂)ₙO(CH₂)ₘO(CH₂)ₖ R⁵, worin n=1,2,3,4 , m=1,2,3,4, k=0,1,2,3,
4-Biphenyl, 1,3-Dioxan-2-ylethyl, 2-Methyl-2-phenylpropyl.

2. Pharmazeutische Zusammensetzung enthaltend eine Verbindung der Formel I als Arzneimittelwirkstoff.

## Revendications

1. Composés de formule I : 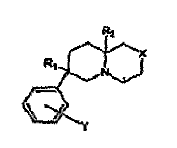
R¹ ; alkyle en C₁-C₆, phényle,
R² : H, alkyle en C₁-C₆, alkylcycloalkyle
X : carbonyle, CR³R⁴, C(OH)R⁶, CR⁶OR⁶
Y: (R³)ₖ où k = 0, 1, 2, 3
où R³ égale :
H, F, Cl, Br, CF₃, alkyle en C₁-C₆, cycloalkyle, phényle,
O(CH₂)ₖR⁵, N (CH₂) ₖR⁵ où k = 0, 1, 2, 3
O(CH₂)ₙO(CH₂)ₖR⁵, où n = 1, 2, 3, 4, k = 0, 1, 2, 3
N(CH₂)ₙO(CH₂)ₖR⁵, où n = 2, 3, 4, k = 0, 1, 2, 3
O(CH₂)ₙO(CH₂)ₘO(CH₂)ₖR⁵, où n = 1, 2, 3, 4, m = 1, 2, 3, 4, k = 0, 1, 2, 3, à l'exclusion de m = n = 1
N(CH₂)ₙO(CH₂)ₘO(CH₂)ₖR⁵, où n = 2, 3, 4, m = 1, 2, 3, 4, k = 0, 1, 2, 3, à l'exclusion de m = n = 1
(CH₂)ₖR⁵, où k = 0, 1, 2, 3
(CH₂)ₙO(CH₂)ₖR⁵, où n = 1, 2, 3, 4, k = 0, 1, 2, 3
(CH₂)ₙO(CH₂)ₘO(CH₂)ₖR⁵, où n = 1, 2, 3, 4, m = 1, 2, 3, 4, k = 0, 1, 2, 3
NHR⁵, N(R⁵)₂, NR⁴(C=O)R⁵, NR⁴(SO₂)R⁵
pipéridyle, pyrrolidinyle, morpholinyle,
où R⁴ égale : H, alkyle en C₁-C₆, cycloalkyle, alkylcycloalkyle
où R⁵ égale :
H, alkyle en C₁-C₆, isopropyle, isobutyle, cyclopentyle, cyclohexyle, cycloheptyle, benzyle, phényle, 4-diphényle, 1,3-dioxan-2-yléthyle, 2-méthyl-2-phénylpropyle, 2-biphényle, 4-biphényle, 2-phénoxyphényle, 4-phénoxyphényle,
où R⁶ égalé :
(CH₂)ₖR⁵, où k = 0, 1, 2, 3
(CH₂)ₙO(CH₂)ₖR⁵, où n = 1, 2, 3, 4, k = 0, 1, 2, 3
(CH₂)ₙO(CH₂)ₘO(CH₂)ₖR⁵, où n = 1, 2, 3, 4, m = 1, 2, 3, 4, k = 0, 1, 2, 3
4-biphényle, 1,3-dioxan-2-yléthyle, 2-méthyl-2-phénylpropyle.

2. Composition pharmaceutique contenant un composé de formule I comme substance médicamenteuse.
